# EUROPÄISCHE PATENTSCHRIFT

(11) **EP 1 151 441 B1**
(45) Veröffentlichungstag und Bekanntmachung des Hinweises auf die Patenterteilung: **28.04.2004**
(21) Anmeldenummer: 00908984.8
(22) Anmeldetag: 07.02.2000
(51) Int. Cl.: G21K 1/04

(54) **KONTURENKOLLIMATOR FÜR DIE STRAHLENTHERAPIE**
CONTOUR COLLIMATOR FOR USE IN RADIOTHERAPY
COLLIMATEUR DE CONTOURS UTILISE EN RADIOTHERAPIE

(30) Priorität: 06.02.1999 DE 19904972
(43) Veröffentlichungstag der Anmeldung: 07.11.2001
(73) Patentinhaber: Deutsches Krebsforschungszentrum Stiftung des öffentlichen Rechts, 69120 Heidelberg (DE)
(72) Erfinder: SCHLEGEL, Wolfgang, D-69121 Heidelberg (DE); PASTYR, Otto, D-69181 Leimen (DE); ECHNER, Gernot, D-69257 Wiesenbach (DE); HÖVER, Karl-Heinz, D-74889 Sinsheim (DE); RICHTER, Jürgen, D-97080 Würzburg (DE)
(74) Vertreter: Castell, Klaus, Dr.-Ing.
(86) Internationale Anmeldenummer: PCT/DE2000/000347
(87) Internationale Veröffentlichungsnummer: WO 2000/046813

(56) Entgegenhaltungen:
- EP-A- 0 314 214
- EP-A- 0 387 921
- EP-A- 0 562 644
- DE-A- 19 536 804
- DE-A- 19 639 861

## Beschreibung

Die Erfindung betrifft einen Konturenkollimator für die Strahlentherapie mit einer Mehrzahl an mittels eines Antriebs relativ zueinander verschiebbar angeordneten Blendenelementen, wobei eine Vielzahl an Blendenelementen jeweils eigene Antriebe zugeordnet sind, die die Blendenelemente in eine Führungsschiene hin und her bewegen.

Ein derartiger Konturenkollimator ist beispielsweise aus der DE 195 36 804.5 A1 bekannt. Bei diesem Konturenkollimator sind einer Vielzahl an Blendenelementen jeweils eigene Antriebe zugeordnet, die die Blendenelemente in einer Führungsschiene hin und her bewegen. Über die Steuerung der einzelnen Blendenelemente wird ein Bestrahlungsfeld festgelegt, mit dem am bestrahlten Körperteil eine spezielle zu bestrahlende Kontur erzeugt werden kann. Dieser Konturenkollimator ist besonders für kleine Bestrahlungsfelder geeignet. Eine Vergrößerung des bekannten Konturenkollimators zur Erzeugung größerer Bestrahlungsfelder scheitert daran, dass die benötigten Motoren zu groß werden und kaum noch um das Bestrahlungsfeld herum anordenbar sind.

Der Erfindung liegt daher die Aufgabe zugrunde, einen bekannten Konturenkollimator so weiter zu entwickeln, dass er auch für größere Bestrahlungsfelder geeignet ist.

Diese Aufgabe wird dadurch gelöst, dass die Blendenelemente nur auf der Seite des Antriebs abgestützt sind.

Der Erfindung liegt die Erkenntnis zugrunde, dass die Blendenelemente sowohl abgestützt als auch geführt werden müssen. Hierfür wird bei dem herkömmlichen Konturenkollimator eine Schiene vorgesehen, die das Gewicht der Blendenelemente aufnimmt und die Blendenelemente parallel zueinander führt.

Insbesondere bei der Verwendung größerer Blendenelemente entsteht jedoch sehr viel Reibung in den Führungsschienen und die Blendenelemente neigen zum Verkanten, wodurch hohe Kräfte benötigt werden, um die Blendenelemente zu bewegen. Die Verwendung größerer Motoren führt jedoch zu großen Abmessungen des Konturenkollimators, unerwünschter, nicht zu akzeptierender Gewichtszunahme und vor allem zu Platzproblemen, da die Motoren möglichst in unmittelbarer Nähe zu den Blendenelementen angeordnet werden sollten.

Die Blendenelemente des erfindungsgemäßen Konturenkollimators sind hingegen nur im Bereich des Antriebs vorzugsweise über ein Festlager abgestützt. Die darüber hinaus notwendigen Führungen für die Blendenelemente dienen allein der Positionierung und nehmen jedoch keine Gewichtskräfte der Blendenelemente auf. Durch die Nähe der Abstützung zum Antrieb wird ein Verkanten der Blendenelemente vermieden, wodurch geringere Kräfte zur Bewegung der Blendenelemente benötigt werden. Dadurch können die Motoren kleiner ausgelegt werden und sind somit auf engstem Raum nebeneinander anordenbar.

Besonders vorteilhaft ist es, wenn die Blendenelemente im Bereich des Antriebs eine Zahnstange aufweisen. Diese Zahnstange erlaubt es, beispielsweise mit einem senkrecht zur Bewegungsrichtung der Blenden angetriebenen Zahnrad zusammenzuwirken, um eine möglichst verlustfreie Kraftübertragung zu erzielen. Außerdem erlaubt die Zahnstange einen sehr kompakten Aufbau des Konturenkollimators, da die Antriebe dadurch sehr eng nebeneinander anordenbar sind.

Vorteilhaft ist es, wenn in unmittelbarer Nähe des Antriebs auch eine Führung für die Blendenelemente angeordnet ist. Die Führung im Bereich des Antriebs gewährleistet ein sicheres Zusammenwirken zwischen Antrieb und Blendenelement, insbesondere beim Zusammenwirken von Zahnstange und Zahnrad gewährleistet die Führung eine sichere Positionierung der Elemente relativ zueinander.

Um die Blendenelemente möglichst reibungslos zu führen wird vorgeschlagen, dass auf der dem Antrieb gegenüberliegenden Seite der Blendenelemente eine lose Lagerung für die Blendenelemente vorgesehen ist. Diese lose Lagerung nimmt nur geringe Seitengewichtskräfte in einer zur Blenden-Bewegungsrichtung senkrechten Ebene auf und sorgt in erster Linie dafür, dass die Blendenelemente im Wesentlichen parallel zueinander geführt werden.

Eine bevorzugte Ausführungsform sieht vor, dass mindestens zwei Blendenelemente in einem Abstand leicht versetzt gegenüberliegend angeordnet sind und um mehr als den halben Anstand aufeinanderzu bewegbar sind. Diese Anordnung ermöglicht einen sogenannten "over travel", der die Ausbildung spezieller Konturen und das Verschränken von gegenüberliegenden Blendenelementen ineinander ermöglicht.

Um den Konturenkollimator optimal an den Strahlengang der Therapiestrahlen anzupassen wird vorgeschlagen, dass die Längsachsen von mindestens zwei Blendenelementen in ihrer Erstreckung zwischen dem Antrieb und ihrer gegenüberliegenden Seite einen Winkel bilden. Dies erlaubt es, die Blendenelemente konisch auszubilden und fächerartig anzuordnen, wobei sich der Fächer in Richtung der verwendeten Strahlen erweitert.

Vorteilhaft ist es, wenn mindestens zwei Blendenelemente in ihrer Erstreckung zwischen dem Antrieb und ihrer gegenüberliegenden Seite die gleiche Länge aufweisen. Vorzugsweise sind sogar alle Blendenelemente im Wesentlichen gleich geformt, um die Kosten für die Herstellung der Blendenelemente zu reduzieren und um defekte Blendenelemente leichter austauschen zu können.

Eine starke Gewichtsreduktion der Blendenelemente wird dadurch erzielt, dass die im Bereich des Antriebs liegende Seite der Blendenelemente in Bewegungsrichtung des Blendenelementes länger ist als dessen gegenüberliegende Seite. Während der Antrieb an der langen Seite des Blendenelementes mit dem Blendenelement zusammenwirkt, ist das Blendenelement nur in dem Bereich, in dem es mit der Strahlung in Berührung kommt, in voller Höhe ausgebildet.

Eine schnelle Anpassung des Kollimators an unterschiedlichste Einsatzzwecke wird dadurch erreicht, dass mindestens zwei vorzugsweise die Hälfte aller Blendenelemente ein Blendenpaket bilden, das zusätzlich zur Bewegung der einzelnen Blendenelemente in Bewegungsrichtung der Blendenelemente verschiebbar angeordnet ist. Dadurch kann die Position des gesamten Blendenpaketes auf einfache Art und Weise verstellt werden. Dadurch kann das Bestrahlungsfeld schnell vergrößert bzw. verkleinert werden.

Dies wird vorzugsweise dadurch erreicht, dass zwei Blendenpakete in Bewegungsrichtung der Blenden gegenüberliegend angeordnet sind und auf Führungsstangen aufeinander ausrichtbar gelagert sind. Dadurch kann der Konturenkollimator beispielsweise durch eng beieinander liegende Blendenpakete mit hohem overtravel betrieben werden. Weit auseinander gezogene Blendenpakete erlauben hingegen die Ausbildung einer besonders großen, konturierten bestrahlten Fläche.

Ein besonders kompakter Aufbau des Konturenkollimators ist dadurch zu erzielen, dass der Antrieb eine senkrecht zum Blendenelement angeordnete, zu einem Motor führende Achse aufweist. Dies erlaubt es, eng nebeneinander viele Motoren zum Antrieb vieler Blendenelemente anzuordnen.

Dadurch wird die Anlage besonders kompakt im Aufbau und gleichzeitig sind die nebeneinander liegenden Motoren leicht kontrollierbar und im Schadensfall leicht austauschbar.

Insbesondere der erfindungsgemäße Aufbau erlaubt es, jedem Blendenelement einen eigenen Antrieb zuzuordnen, wodurch eine individuelle Konfiguration der Position der Blendenelemente erzeugt werden kann.

Um die Position der Blendenelemente zur Überprüfung und Dokumentation einer Datenverarbeitungsanlage zuzuführen, wird vorgeschlagen, dass jeder Antrieb ein Drehpotentiometer aufweist, der auf engstem Raum angebracht ist oder parallel zu den Blendenelementen angeordnete Linearpotentiometer oder andere Linearmeßsysteme wie beispielsweise induktive oder optische Systeme verwendet werden.

Ein bevorzugtes Ausführungsbeispiel eines erfindungsgemäßen Konturenkollimators ist in der Zeichnung dargestellt und wird im Folgenden näher erläutert. Es zeigt
- Figur 1: eine dreidimensionale Ansicht des Konturenkollimators, mit exemplarisch eingezeichneten Blendenelementen,
- Figur 2: eine schematische Ansicht des Konturenkollimators aus Figur 1 von dessen Stirnseite,
- Figur 3: einen vergrößerten Ausschnitt aus Figur 2,
- Figur 4: eine schematische Aufsicht auf den Konturenkollimator aus Figur 1,
- Figur 5: die Ansicht einer Antriebseinheit und
- Figur 6: eine vergrößerte Einzelheit aus Figur 3
- Figur 7: eine Führungsplatte für die Blendenelemente
- Figur 8: eine alternative Ausführungsform einer Führungsplatte mit eingesetzten Blendenelementen und
- Figur 9: einen vergrößerten Ausschnitt aus Figur 8 mit nur einem Blendenelement.

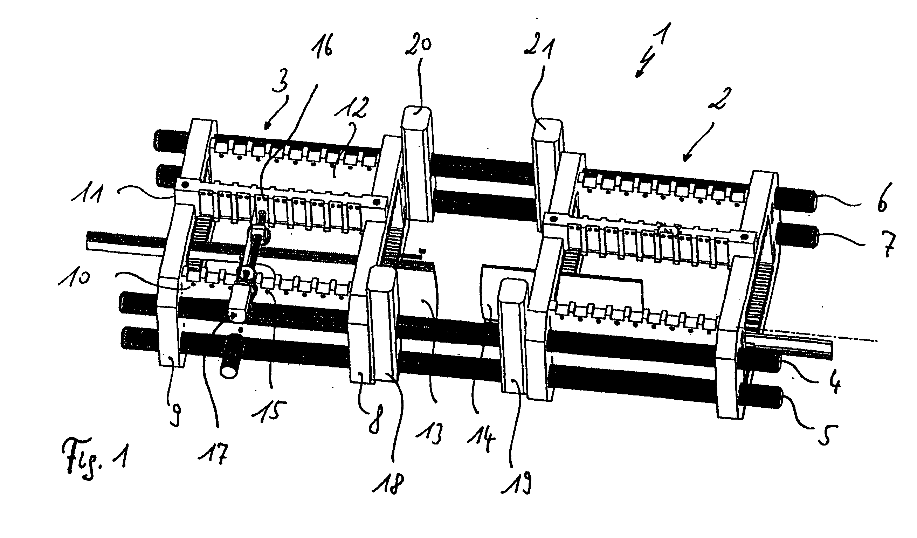

Der in Figur 1 gezeigt Konturenkollimator 1 besteht aus einem rechten 2 und aus einem linken Lamellenblock 3, die relativ zueinander verschiebbar auf vier Stangen 4, 5, 6, 7 angeordnet sind.

Jeder Lamellenblock besteht aus einer vorderen Platte 8 und einer hinteren Platte 9, die durch Stützstege 10, 11, 12 beabstandet zueinander gehalten werden. Die Platten weisen Einrichtungen auf, mit denen Blendenelemente 13, 14 abgestützt und geführt werden. Die senkrecht zu den Platten angeordneten Stege 10, 11, 12 weisen Bohrungen 15 und Nuten 16 auf, mittels derer Antriebseinheiten 17 am Lamellenblock befestigt werden.

Die die Lamellenblöcke 2, 3 tragenden Stangen 4 bis 7 sind an durchbohrten Stäben 18, 19, 20, 21 befestigt und die Stäbe dienen dem Anbringen des gesamten Konturenkollimators 1 an eine Platte, die verschiebbar an einer Strahlenvorrichtung, die in der Abbildung nicht gezeigt ist, befestigt ist.

Zur Erhöhung der Übersichtlichkeit sind in Figur 1 nur ein hängendes 13 und ein stehendes 14 Blendenelement sowie eine Antriebseinheit 17 dargestellt. Der Konturenkollimator weist jedoch eine Vielzahl an parallel zueinander angeordneten Blendenelementen auf, denen jeweils eine eigene Antriebseinheit 17 zugeordnet ist.

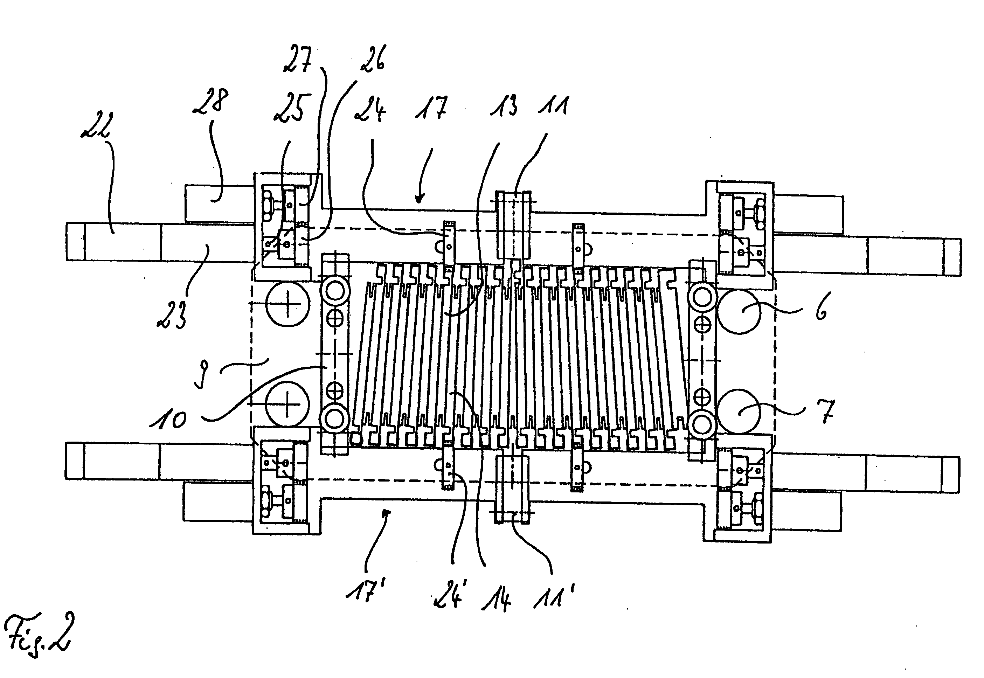

Die Anordnung der Vielzahl an Blendenelementen ist der Figur 2 zu entnehmen, in der sämtliche Blendenelemente eingezeichnet sind.

Die Blendenelemente sind leicht halbkreisförmig angeordnet und jedes zweite Blendenelement wird aus Platzgründen oben angetrieben und die dazwischen liegenden Blendenelemente werden unten angetrieben.

Für den Antrieb der Blendenelemente ist pro Blendenelement 13 eine Antriebseinheit 17 vorgesehen. Diese Antriebseinheit besteht aus einem Motor 22, einem Getriebe 23 und einem Antriebszahnrad 24. Getriebe 23 und Zahnrad 24 sind über eine Welle 25 miteinander verbunden, auf der ein Zahnrad 26 angeordnet ist, das mit einem Zahnrad 27 zusammen wirkt und ein Drehpotentiometer 28 proportional zur Position des Blendenelementes 13 verstellt. Das danebenliegende Blendenelement 14 wird von einem darunterliegenden Antriebszahnrad 24' angetrieben, das eine entsprechende Antriebseinheit 17' aufweist. Die Antriebseinheiten 17 und 17' sind an den Stegen 10 und 11, 11' befestigt und diese Stege sind mittels der Platten 8 und 9 an den Stangen 4 bis 7 befestigt.

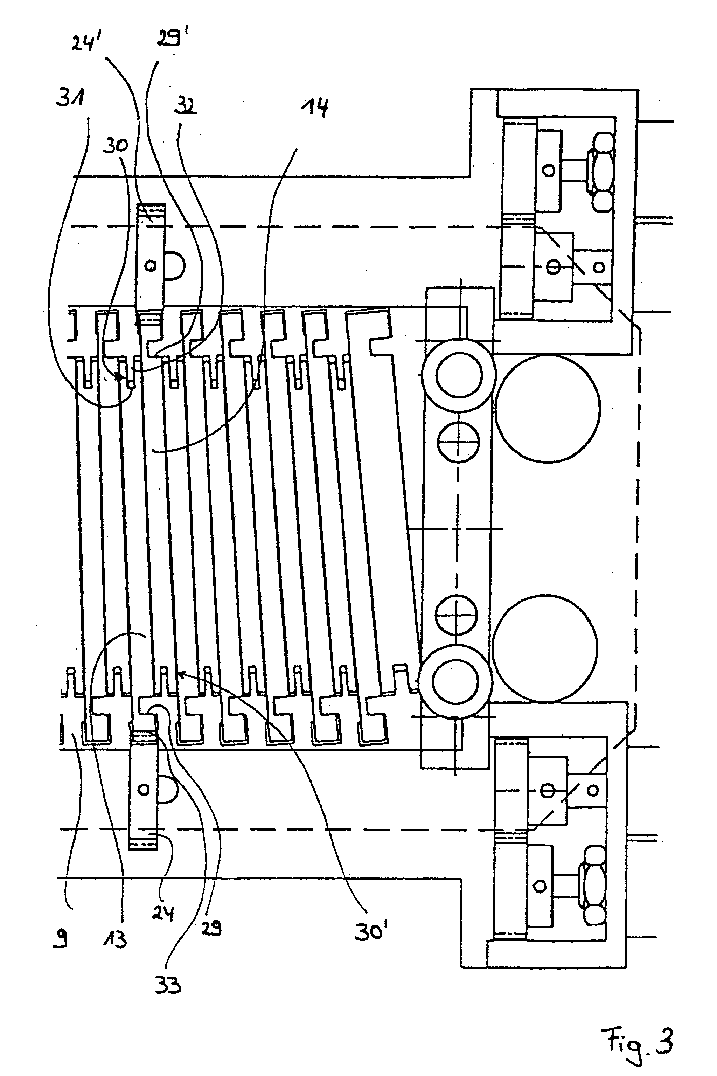

Die Aufhängung eines Blendenelementes ist in Figur 3 deutlicher herausgezeichnet. Das gesamte Gewicht des Blendenelementes 13 liegt auf dem Steg 29, der direkt dem Antriebsrad 24 gegenüberliegend angeordnet ist. Die übrigen Flächen der Platte 9, die mit dem Blendenelement 13 in Berührung treten, dienen ausschließlich der Führung, damit das Blendenelement 13 nicht von der Auflagefläche 29 abrutscht. Die zwischen dem Blendenelemente 13 und der Platte 9 gebildeten Führungsflächen wirken jedoch mit einer losen Lagerung 30 am gegenüberliegenden Plattenende zusammen. Für diese lose Lagerung 30 ist in das Blendenelement 13 eine Nut 31 eingefräst, die einen Führungszapfen 32 der Platte 9 aufnimmt.

Das danebenliegende Blendenelement 14 hat eine Auflagefläche 29', die dem Antriebzahnrad 24' gegenüberliegend angeordnet ist und die Gewichtskraft der Platte aufnimmt. Entsprechend ist das Blendenelement 14 in einer dem Antriebzahnrad 24' gegenüberliegenden losen Loslager 30' geführt.

Beim Antrieb beispielsweise des Antriebzahnrads 24 wird somit über die Zahnstange 33 das Blendenelement 13 verschoben. Um eine minimale Reibung zu gewährleisten, gleitet das Blendenelement 13 auf der Auflagefläche 29, während es zusätzlich durch das gegenüberliegende Loslager 30 geführt wird. Entsprechend liegt das Blendenelement 14 nicht auf dem Antriebsrad 24, sondern auf der Auflagefläche 29' auf, während die räumliche Führung vom Loslager 30' übernommen wird.

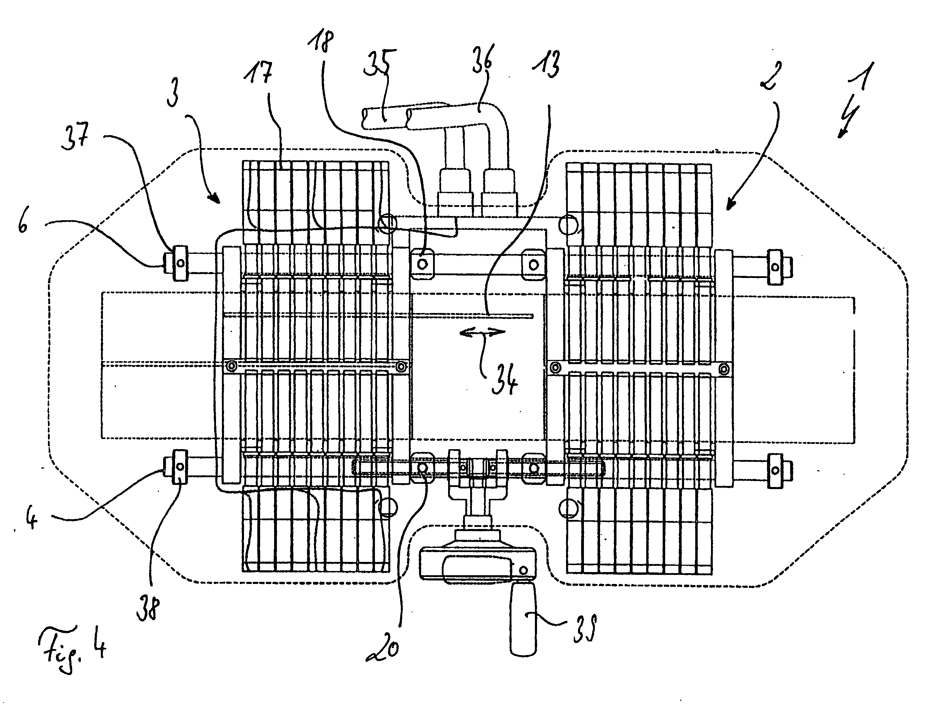

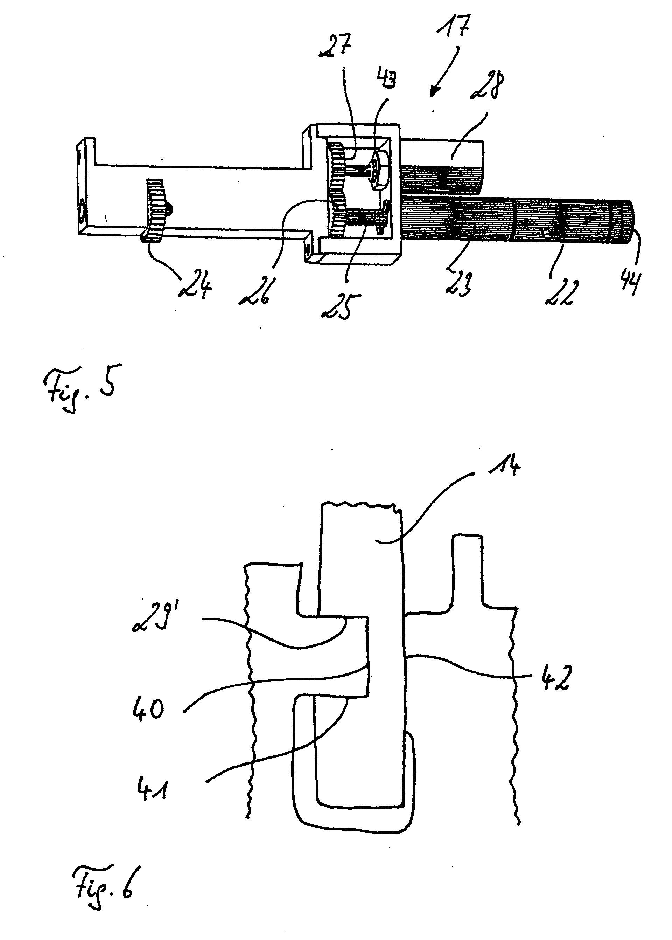

Die in Figur 4 gezeigte schematische Aufsicht auf den Konturenkollimator 1 zeigt nur als Beispiel das Blendenelement 13, das in Richtung des Pfeiles 34 beweglich angeordnet ist. Der Antrieb des Blendenelements 13 erfolgt über die Antriebseinheit 17, die ihre Energie - wie die anderen Antriebseinheiten - über die Anschlußkabel 35, 36 erhält. Das Blendenelement 13 ist ein Blendenelement aus dem linken Lamellenblock 3, der auf den Stangen 4 und 6 und den darunterliegenden Stangen 5 und 7 (in Figur 5 nicht gezeigt) verschiebbar gelagert ist. Auch der Lamellenblock 3 ist in Richtung des Pfeiles 34 verschiebbar, wobei der Verschiebebereich durch äußere Anschläge 37, 38 und die Stäbe 18 und 20 begrenzt ist. Die Verschiebung der Lamellenblöcke 2 und 3 erfolgt über ein Handrad 39, mit dem die Lamellenblöcke 2 und 3 symmetrisch zu einer Mittellinie auseinander- und zusammengefahren werden können. Alternativ können die Lamellenblöcke 2 und 3 mittels eines oder zweier Antriebe motorisch einzeln oder gemeinsam verfahren werden.

Figur 5 zeigt noch einmal als dreidimensionale Darstellung eine Antriebseinheit 17. Der Motor 22 ist an einem Getriebe 23 angeordnet, das über die Welle 25 ein Antriebszahnrad 24 antreibt. Außerdem ist auf der Welle 25 ein weiteres Zahnrad 26 befestigt, das mit dem Zahnrad 27 zusammenwirkt. Das Zahnrad 27 wirkt wiederum über eine Welle 40 auf ein Drehpotentiometer 28. Das Drehpotentiometer 28 gibt einen analogen Wert an eine Steuerung (nicht gezeigt) weiter, der der Position des Blendenelementes 13 innerhalb des Konturenkollimators entspricht.

Anstelle des Drehpotentiometers 28 kann ebenso ein Resolver angeordnet werden, der pro Umdrehung eine vorbestimmte Anzahl an Impulsen abgibt, um digital einen Wert für die Lamellenposition der Steuerungseinrichtung zu melden.

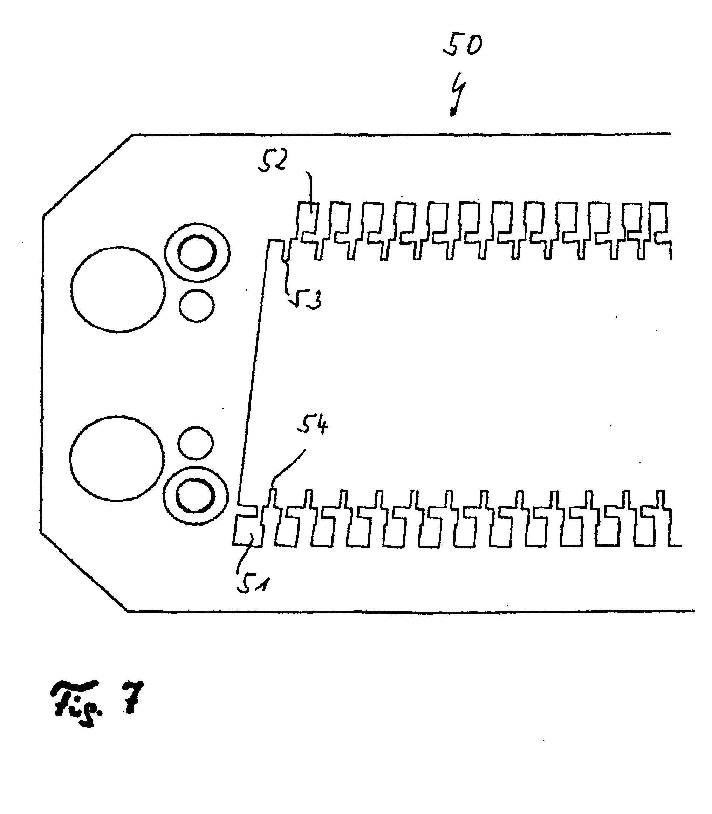

Die untere Führung des Blendenelements 14 ist in Figur 6 vergrößert dargestellt. Während das Blendelement 14 auf der Fläche 29' aufliegt, dienen die Berührungsflächen 40, 41 und 42 als Führung und bilden mit der Fläche 29' ein Festlager.

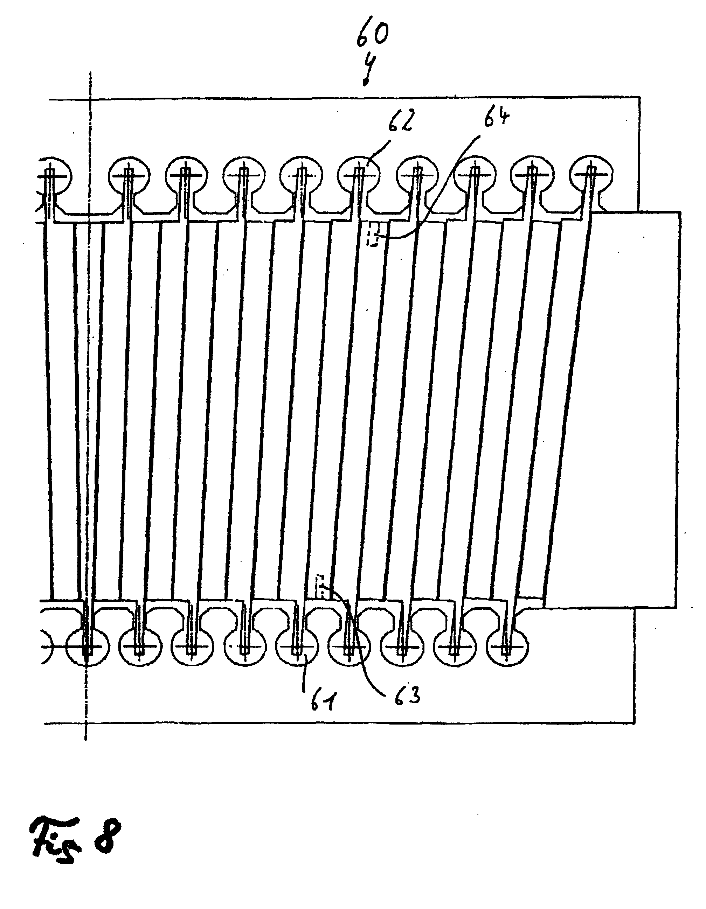

Die in Figur 7 gezeigte Führungsplatte 50 zeigt deutlich die spezielle Form der Einschnitte 51, 52 und der jeweils gegenüberliegenden Zapfen 53, 54. Die kompakte Anordnung von als Stützlager dienendem Einschnitt 51, 52 und als lose Lagerung dienenden Zapfen 53, 54 ermöglicht die hochpräzise Positionierung der Blendenelemente. Die Führungsplatte ist durch Drahterosion hergestellt. Dieses Verfahren ist preiswert, schnell und vor allem extrem genau durchführbar.

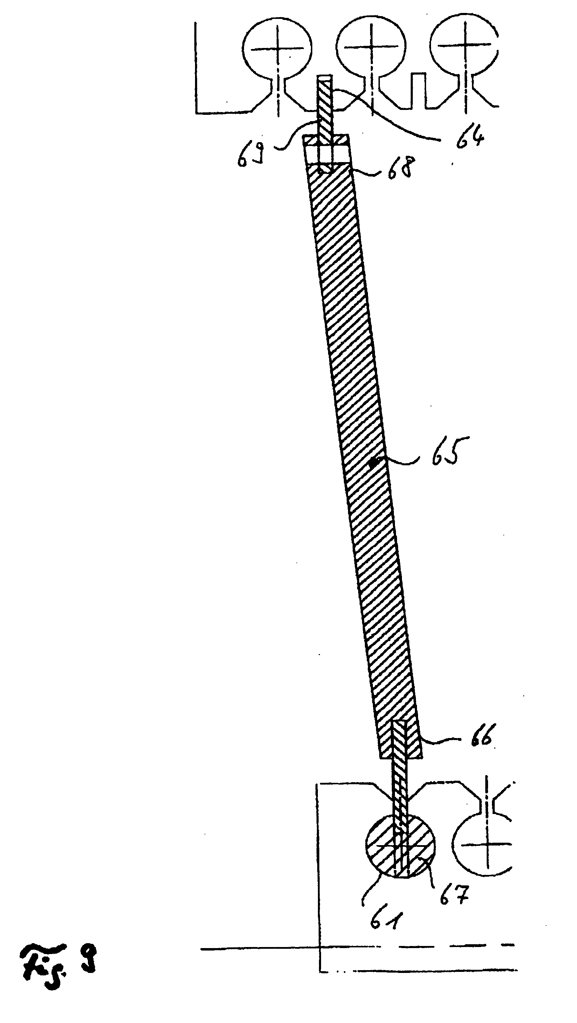

Das erfindungsgemäße Prinzip ist nicht auf die bisher beschriebene Ausführungsförm beschränkt sondern kann auf verschiedenartigste Weise verwirklicht werden. Nur beispielhaft ist daher in Figur 8 eine Ausführungsform mit einer Führungsplatte 60 dargestellt, in der runde Einschnitte 61, 62 angebracht sind. Diese runden Einschnitte 61, 62 dienen als Stützlager und wirken mit gegenüber angeordneten Nuten 63, 64 zusammen, die als lose Lagerung dienen.

Der in Figur 9 gezeigte Ausschnitt zeigt deutlicher, wie ein Blendenelement 65 zwischen einem Stützlager 61 und einer losen Lagerung 63 abgeordnet ist. Das Blendenelement 65 weist hierzu an seinem Ende einen kopfartigen Fortsatz 67 und an seinem Ende 68 eine federartigen Fortsatz 69 auf. Der kopfartige Fortsatz 67 ist im runden Einschnitt 61 abgestützt und an dieser Seite greift der Antrieb (nicht gezeigt) an. Der federartige Fortsatz 69 greift in die Nut 64 ein, die größer als der Fortsatz ausgebildet ist, um in Längsrichtung Toleranzen auszugleichen. Auch diese Ausführungsform ist durch Drahterosion einfach herstellbar.

## Patentansprüche

1. Konturenkollimator (1) für die Strahlentherapie mit einer Mehrzahl an mittels eines Antriebs (17, 17') relativ zueinander verschiebbar angeordneten Blendenelementen (13, 14), wobei eine Vielzahl an Blendenelementen (13,14) jeweils eigene Antriebe (17, 17') zugeordnet sind, die die Blendenelemente (13, 14) in einer Führungsschiene hin und her bewegen ***dadurch gekennzeichnet, dass*** die Blendenelemente (13, 14) nur auf der Seite des Antriebs (17, 17') abgestützt sind.

2. Konturenkollimator nach Anspruch 1, ***dadurch gekennzeichnet, dass*** die Blendenelemente (13, 14) im Bereich des Antriebs (17, 17') eine Zahnstange (33) aufweisen.

3. Konturenkollimator nach einem der vorhergehenden Ansprüche, ***dadurch gekennzeichnet, dass*** in unmittelbarer Nähe des Antriebs (17, 17') eine Führung für die Blendenelemente (13, 14) angeordnet ist.

4. Konturenkollimator nach einem der vorhergehenden Ansprüche, ***dadurch gekennzeichnet, dass*** auf der dem Antrieb (17, 17') gegen-überliegenden Seite der Blendenelemente (13, 14) eine lose Lagerung (30, 30') für die Blendenelemente (13, 14) vorgesehen ist.

5. Konturenkollimator nach einem der vorhergehenden Ansprüche, ***dadurch gekennzeichnet, dass*** mindestens zwei Blendenelemente (13, 14) in einem Abstand, leicht versetzt gegenüberliegend angeordnet sind und um mehr als den halben Abstand ordnet sind und um mehr als den halben Abstand aufeinanderzu bewegbar sind.

6. Konturenkollimator nach einem der vorgehenden Ansprüche, ***dadurch gekennzeichnet, dass*** die Längsachsen von mindestens zwei Blendenelementen (13, 14) in ihrer Erstreckung zwischen dem Antrieb (17, 17') und ihrer gegenüberliegenden Seite einen Winkel bilden.

7. Konturenkollimator nach einem der vorhergehenden Ansprüche, ***dadurch gekennzeichnet, dass*** die im Bereich des Antriebs (17, 17') liegende Seite eines Blendenelementes (13, 14) in Bewegungsrichtung (34) des Blendenelementes (13, 14) länger ist als dessen gegenüberliegende Seite.

8. Konturenkollimator nach einem der vorhergehenden Ansprüche, ***dadurch gekennzeichnet, dass*** mindestens zwei Blendenelemente (13, 14) ein Bleridenpaket (2, 3) bilden, das zusätzlich zur Bewegung der einzelnen Blendenelemente (13, 14) in Bewegungsrichtung (34) der Blendenelemente (13, 14) verschiebbar angeordnet ist.

9. Konturenkollimator nach Anspruch 8, ***dadurch gekennzeichnet*, *dass*** zwei Blendenpakete (2, 3) in Bewegungsrichtung (34) der Blendenelemente (13, 14) gegenüberliegend angeordnet sind und auf Führungsstangen (4, 5, 6, 7) aufeinander ausrichtbar gelagert sind.

10. Konturenkollimator nach einem der vorhergehenden Ansprüche, ***dadurch gekennzeichnet, dass*** der Antrieb (17, 17') ein Drehpotentiometer (28) zur Erfassung der Blendenposition aufweist.

## Claims

1. A contour collimator (1) for radiotherapy with a plurality of diaphragm elements (13, 14) that are disposed so as to be slidable relative to each other by means of a drive (17, 17'), a great number of diaphragm elements (13, 14) being each assigned its own drive (17, 17') that reciprocates the diaphragm elements (13, 14) within a guide rail, ***characterized in that*** the diaphragm elements (13, 14) are only supported on the side of the drive (17, 17').

2. The contour collimator according to claim 1, ***characterized in that*** the diaphragm elements (13, 14) comprise a rack (33) in the region of the drive (17, 17').

3. The contour collimator according to one of the aforementionned claims, ***characterized in that*** a guide for the diaphragm elements (13, 14) is to be disposed in immediate proximity to the drive (17, 17').

4. The contour collimator according to one of the aforementionned claims, ***characterized in that,*** on the side ofthe diaphragm elements (13, 14) that is opposed to the drive (17, 17'), there is provided a lose bearing (30, 30') for the diaphragm elements (13, 14).

5. The contour collimator according to one of the aforementionned claims, ***characterized in that* at** least two diaphragm elements (13, 14) are oppositely disposed from each other in a spaced-apart and slightly offset configuration and can be moved towards each other by more than half their spacing.

6. The contour collimator according to one of the aforementionned claims, ***characterized in that*** the longitudinal axes, which extend between the drive (17, 17'), of at least two diaphragm elements (13, 14) are inclined at an angle.

7. The contour collimator according to one of the aforementionned claims, ***characterized in that****,* in the direction of motion (34) of the diaphragm elements (13, 14), the side of a diaphragm element (13, 14) that lies in the region of the drive (17, 17') is longer than its opposite side.

8. The contour collimator according to one of the aforementionned claims, ***characterized in that*** at least two diaphragm elements (13, 14) form a diaphragm stack (2, 3) that is disposed so as to be slidable in the direction of motion (34) of the diaphragm elements (13, 14), additionally to the motion of every single diaphragm element (13, 14).

9. The contour collimator according to claim 8, ***characterized in that*** two diaphragm stacks (2, 3) are oppositely disposed in the direction of motion (34) of the diaphragm elements (13, 14) and are alignably carried on guide bars(4, 5, 6, 7).

10. The contour collimator according to one of the aforementionned claims, ***characterized in that*** the drive (17, 17') comprises a rotary potentiometer (28) for detecting the position of the diaphragms.

## Revendications

1. Collimateur de conformation (1) utilisé en radiothérapie avec une pluralité d'éléments diaphragme (13, 14) disposés de manière à pouvoir être déplacés les uns par rapport aux autres par un organe de commande (17, 17'), un grand nombre d' éléments diaphragme (13, 14) chacun étant assignés à son propre organe de commande (17, 17') qui imprime aux éléments diaphragme (13, 14) un mouvement de va-et-vient dans un rail de guidage, ***caractérisé en ce que*** les éléments diaphragme (13, 14) ne sont en appui que du côté de l'organe de commande (17, 17').

2. Collimateur de conformation selon la revendication 1, ***caractérisé en ce que*** les éléments diaphragme (13, 14) comportent une crémaillère (33) dans la région de l'organe de commande (17, 17')

3. Collimateur de conformation selon l'une quelconque des revendications précédentes, ***caractérisé en ce qu'***un guidage pour les éléments diaphragme (13, 14) est disposé dans le voisinage immédiat de l'organe de commande (17, 17').

4. Collimateur de conformation selon l'une quelconque des revendications précédentes, ***caractérisé en ce qu*'**un palier libre (30, 30') est prévu pour les éléments diaphragme (13, 14) du côté des éléments diaphragme (13, 14) opposé à l'organe de commande (17, 17').

5. Collimateur de conformation selon l'une quelconque des revendications précédentes, ***caractérisé en ce qu'***au moins deux éléments diaphragme (13, 14) sont disposés à distance, en léger décalage et en regard l'un de l'autre et peuvent être rapprochés l'un de l'autre de plus de la moitié de la distance qui les sépare.

6. Collimateur de conformation selon l'une quelconque des revendications précédentes, ***caractérisé en ce que*** les axes longitudinaux d'au moins deux éléments diaphragme (13, 14) forment un angle par le biais de leur extension entre l'organe de commande (17, 17') et leur côté opposé.

7. Collimateur de conformation selon l'une quelconque des revendications précédentes, **caractérisé en ce que** le côté d'un élément diaphragme (13, 14) situé dans la région de l'organe de commande (17, 17') est plus long dans le sens du mouvement (34) de l'élément diaphragme (13, 14) que son côté opposé.

8. Collimateur de conformation selon l'une quelconque des revendications précédentes, **caractérisé en ce qu**' au moins deux éléments de diaphragme (13, 14) forment un paquet de diaphragmes (2, 3) qui, en plus du mouvement de chacun des éléments diaphragme (13, 14), est monté à coulissement dans le sens du mouvement (34) des éléments diaphragme (13, 14).

9. Collimateur de conformation selon la revendication 8, **caractérisé en ce que** deux paquets de diaphragmes (2, 3) sont disposés en regard l'un de l'autre dans le sens du mouvement (34) des éléments de diaphragme (13, 14) et qu'ils sont supportés sur des barres de guidage (4, 5, 6, 7) de manière à pouvoir être alignés l'un sur l'autre.

10. Collimateur de conformation selon l'une quelconque des revendications précédentes, **caractérisé en ce que** l'organe de commande (17, 17') comporte un potentiomètre rotatif (28) pour détecter la position des diaphragmes.
